(19) Europäisches Patentamt
European Patent Office
Office européen des brevets

(11) EP 3 454 829 B1

(12) **EUROPEAN PATENT SPECIFICATION**

(45) Date of publication and mention of the grant of the patent:
**03.11.2021 Bulletin 2021/44**

(21) Application number: **16701547.8**

(22) Date of filing: **27.01.2016**

(51) Int Cl.:
***A61K 8/895*** *(2006.01)* ***A61K 8/31*** *(2006.01)*
***A61K 8/58*** *(2006.01)* ***A61K 8/81*** *(2006.01)*
***A61K 8/891*** *(2006.01)* ***A61Q 5/00*** *(2006.01)*

(86) International application number:
**PCT/EP2016/051639**

(87) International publication number:
**WO 2017/129234 (03.08.2017 Gazette 2017/31)**

(54) **COMPOSITION FOR REDUCTION OF HAIR SPLIT ENDS, USE AND PROCESS THEREOF**

ZUSAMMENSETZUNG ZUR VERRINGERUNG VON GESPALTENEN HAARSPITZEN, VERWENDUNG UND VERFAHREN DAFÜR

COMPOSITION DE RÉDUCTION DES POINTES DE CHEVEUX FOURCHUES, UTILISATIONS ET PROCÉDÉS DE CELLE-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**20.03.2019 Bulletin 2019/12**

(73) Proprietor: **Kao Germany GmbH**
**64297 Darmstadt (DE)**

(72) Inventors:
- **LIPINSKI, Normen**
**64297 Darmstadt / Hessen (DE)**
- **EBNER, Frank**
**64297 Darmstadt / Hessen (DE)**

(74) Representative: **Grit, Mustafa**
**Kao Germany GmbH**
**Pfungstädter Strasse 98-100**
**64297 Darmstadt (DE)**

(56) References cited:
**EP-A1- 3 009 466** **WO-A1-2013/065766**
**WO-A1-2015/001069** **FR-A1- 2 957 253**
**US-A1- 2011 311 471** **US-A1- 2015 232 601**

EP 3 454 829 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a composition and process to treat hair split ends, especially human hair. It also relates to the use of the composition to reduce hair split ends. Additionally the invention is on a kit of parts comprising the composition.

Daily styling routines as well as chemical services such as bleaching, coloring and perming may cause serious hair damage, which may finally result in split ends. The products available are neither able to repair/rebind the split hair tips nor show an effect which sustains a hair wash. This requires frequent application of the composition preferably after every hair wash which is time consuming and not economical for the consumers. There is a great need for new products which effectively repair the split ends and/or prevents the formation of split ends.

The present invention starts from the above problems and aims at providing a composition for repairing and/or preventing formation of split ends which lasts at least for several hair washes.

The inventors of the present invention have unexpectedly found that an anhydrous composition comprising a silicone vinyl dendrimer having at least one group reacting via free radical polymerization, one or more alkanes, one or more copolymers consisting of olefin and aromatic monomers and one or more homopolymeric olefins repairs the split ends and the effect lasts for several hair washes. This relieves the consumer from having to cut the hair only for the reason of split ends. Consequently customers can grow their hair to the desired length.

Silicone vinyl dendrimers are known from EP 0963751 (Dow Corning Inc.). EP 1862162 discloses vinylated silicones for the purpose of skin make-up to confer color and transfer resistance. In US 2013/0224130 and Research Disclosure 495005 anhydrous lip stick compositions are disclosed which comprise vinyl silicone dendrimers. However, the publications are silent on the core of the present invention. EP 3009466 and WO 2013/65766 disclose polymerisable silicone dendrimers in the context of hair conditioning compositions.

Therefore, the first object of the present invention is an anhydrous cosmetic composition comprising:

a) one or more silicone dendrimers with at least one group that reacts via free radical polymerization,

b) One or more monomeric alkanes with a carbon chain length of at least $C_{10}$ or a siloxane,

c) One or more homopolymeric olefins, and

d) One or more copolymers consisting of olefin and aromatic monomers.

The second object is the use of the anhydrous composition to reduce split ends of hair.

The third object is a process of hair treatment comprising the steps of:

i) Optionally washing the hair with a cleansing composition,

ii) Applying the anhydrous cosmetic composition of the present invention,

iii) Optionally elevating the temperature of the hair ranging from 45°C to 90°C for less than 1 min, preferably from 10 to 30 s, wherein the temperature is measured at the surface of the hair fibers,

iv) Optionally rinsing the hair with water.

The fourth object is a kit of parts comprising the anhydrous composition of the present invention.

The vinylated silicone dendrimers according to the present invention possess at least one group that reacts via free radical polymerization. Such groups are acrylic, methacrylic, and vinyl groups. The silicone acrylate dendrimers possess at least one of the groups per dendrimer molecule.

Monoacrlyated dendrimers are available from JNC America under the trade names Silaplane FM 0711, Silaplane FM 0721, Silaplane FM 0725, Silaplane TM 0701, and Silaplane TM 0701T. Bifunctional silicon acrylate dendrimers are also available from JNC America under the trade names Silaplane FM 7711, Silaplane FM 7721, and Silaplane FM 7725.

Vinylated silicon dendrimers according to the present invention are disclosed in EP1055674. Multi-vinylated dendrimers are synthesized by Kim and Hong (Molecules, 2009, Vol. 14, p. 3719-3730).

The most preferred are monoacrylated silicone dendrimers that are sold by Dow Corning under the name DC FA 4002 ID, DC FA 4001 CM, and DC FA 4003 DM.

The total concentration by weight for vinylated silicon dendrimers range from 0.01% to 10%, preferably from 0.1% to 5%, more preferably from 0.15% to 2.5%, most preferably from 0.2% to 2%, by weight calculated to the total of the composition.

Monomeric alkanes with a chain length of at least $C_{10}$ according to the present invention can be either branched, or linear, saturated, or unsaturated with one or more carbon to carbon double bond, whereas the chain length refers to the total number of carbon atoms within the molecule. Non-limiting examples of such compounds are decane, isodecane, undecane, dodecane, isododecane, tridecane, tetradecane. Praticularly preferred are branched alkanes, whereas the most preferred branched alkane is isododecane. The monomeric alkanes are comprised in the composition at a total concentration by weight from 1% to 40%, preferably 1% to 20%, calculated to the total of the composition.

As an alternative to monomeric alkanes, silicones are used according to the present invention. Suitable silicones are cyclic or linear. Suitable cyclic silicones are octamethylcyclotetrasiloxane and decamethylcyclopentasiloxane. The more preferred cyclic silicone is decamethylcyclopentasiloxane. Suitable linear silicones are dimethicone or trimethicone. The more preferred linear silicone is dimethicone. The silicones are comprised in the composition at a total concentration by weight from 0.1% to 40%, preferably 0.1% to 20%, and more preferably 1.0% to 15%, calculated to the total of the composition. It is also possible to use mixtures of monomeric alkanes and cyclic and/or linear silicones. In case of mixtures the total concentration by weight of the mixture is from 1% to 30%, preferably 1% to 20% calculated to the total of the composition whereas the mixing ratio of the compounds may range from 1:100 to 100:1, preferably 50:1 to 1:50, more preferably 20:1 to 1:20 and most preferably 10:1 to 1:10.

The homopolymeric olefin according to the present invention is selected from monomers such as ethene, propene, isopropene, butene, isobutene, pentene, isopentene, hexane, and isohexene. The preferred olefinic homopolymer is polyisobutene. The preferred total concentration by weight of the olefinic homopolymer is from 50% to 90%, calculated to the total of the composition.

Copolymers consisting of olefinic and aromatic monomers according to the present invention comprise olefinic monomers selected from ethylene, propylene, butylene, pentylene, and hexylene and styrene as the aromatic monomer. The copolymer can be copolymerized from one or more of aforementioned olefinic monomers as a starting material with styrene. Furthermore, the composition can comprise one or more of such copolymers. Examples are Ethylene/Propylene/Styrene Copolymer and Butylene/Ethylene/Styrene Copolymer. Copolymers are comprised at a total concentration by weight from 0.01% to 7.5%, preferably from 0.1% to 5%, more preferably from 0.25% to 2.5%, calculated to the total of the composition.

The compositions may further comprise lipophilic ingredients such as vegetable oils, for example, jojoba oil, avocado oil, sunflower seed oil, walnut oil, peanut oil, olive oil, rapeseed oil, cottonseed oil, palm oil, sesame oil, soybean oil, coconut oil, safflower oil, almond oil, macadamia nut oil, grapefruit seed oil, lemon kernel oil, orange kernel oil, apricot kernel oil, castor oil, or any other; liquid paraffins, especially paraffinum perliquidum and paraffinum subliquidum; other silicones such as dimethicones, dimethiconols, aminated silicones with primary, secondary, tertiary or quaternary ammonium groups such as amodimethicone, polysilicone 9, polysilicone 28, and quaternium 80, arylated silicones such as phenyl trimethicone; fatty acid esters such as octyl palmitate, isocetyl palmitate, isopropyl palmitate and octyl stearate, C10- to C36-fatty acid triglycerides, as well as their mixtures. Total concentration of these lipophilic compounds is in the range of 0.1 to 20% by weight, preferably from 1 to 15% by weight, and more preferably from 2 to 10% by weight, calculated to total of the composition.

The composition may comprise one or more ceramide and pseudo-ceramide compound, such as the one according to general formula:

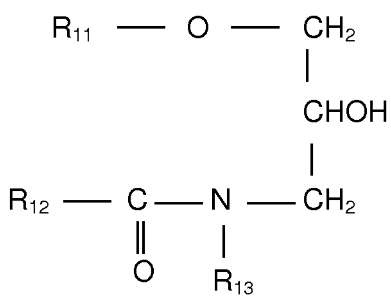

where R11 and R12 are independent from each other alkyl- or. alkenyl group with 10 to 22 carbon atoms, R13 is alkyl or hydroxyl alkyl with 1 to 4 carbon atoms group and n is a number between 1 to 6, preferably 2 or 3. Preferred compound according to the above chemical structure is cetyl-PG-hydroxyethylpalmitamide. Concentration of ceramide type of compounds ranges from 0.01 to 2%, preferably 0.01 to 1% by weight calculated to total the composition.

The composition may comprise antioxidants such as ubiquinone of the formula:

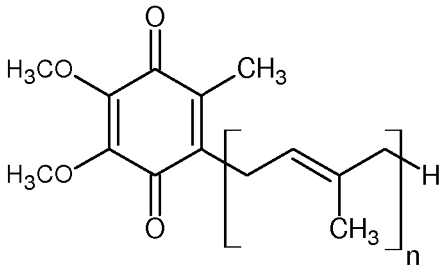

wherein n is a number from 1 to 10. The total concentration of ubiquinone can vary between 0.001 % and 10 % by weight, calculated to the total of the composition.

The composition may comprise one or more organic solvent such as 2-phenoxyethanol, benzyl alcohol, 2-phenylethanol and 2-benzyloxyethanol. Suitable aliphatic alcohols are ethanol, isopropanol, propanol, n-butanol, isobutanol, t-butanol and 1-pentanol. Total concentration of one or more organic solvent is in the range of 0.1 to 15%, preferably 0.5 to 12.5% and more preferably 1 to 10% and most preferably 1 to 7.5% by weight calculated to the total of each composition.

The anhydrous composition of the present invention may further comprise one or more UV filters which may be selected from oils soluble ones. Non-limiting examples are 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2,2',4,4'-tetrahydroxy- benzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15. The total UV filter concentration may be in the range of 0.01 to 1 % by weight calculated to the total composition.

The composition of the present invention may further comprise oil soluble hair direct dyes. Such hair dyes, for example, are selected from CI 12100, CI 12140, CI 12700, CI 12740, CI 26100, CI 26105, CI 45396, CI 45410, CI 45425, CI 47000, CI 60725, CI 60724, CI 81520, and CI 61565. Hair dye concentration may be in the range from 0.001% to 2.0% calculated to the total of the composition.

The composition may further comprise cosmetically acceptable preservatives and fragrances.

The composition of the present invention may be transparent as analysed by the naked eye in a transparent glass container at a solution thickness of 1 cm or by photometric measurements, and may be turbid measured by aforementioned methods. For the appearance and stability of the composition the miscibility of individual optional compounds needs to be considered by the skilled worker prior to its preparation.

## EXAMPLES

The examples are provided to illustrate the invention, but not to limit it.

### Example 1

Hydrogenated polyisobutene and isododecane were mixed in a vessel until homogeneity was reached. In a next step, the Dow Corning DC FA 4002 ID copolymer was added for the inventive example, then the ethylene/propylene/styrene copolymer was added and the mixture was stirred until homogeneity was reached. In a final step fragrance was added.

| Ingredients | Inventive % by weight | Comparative % by weight |
|---|---|---|
| Isododecane | 20.0 | 20.0 |
| DC FA 4002 ID (INCI: isododecane and a crylates/polytrim ethylsil oxymetha cryl ate copolymer) | 3.5 | - |
| Ethylene/Propylene/Styrene Copolymer | 2.0 | 2.0 |

(continued)

| Ingredients | Inventive % by weight | Comparative % by weight |
|---|---|---|
| Butylene/Ethylene/Styrene Copolymer | 2.0 | 2.0 |
| Fragrance | 0.5 | 0.5 |
| Hydrogenated Polyisobutene | Ad 100.00 | Ad 100.00 |

Split end damaged human hair streaks from Caucasian donors were analysed on their hair split ends extend by light microscopy with a 10x to 20x magnification. 10 hair fibres from each hair streak were analysed. The hair length was between 3 and 5 cm per fibre. The hair was treated with the compositions and the immediate effect was measured by light microscopy and expressed as a ratio of hair with closed ends to the total number of hair fibers by the following equation:

$$Hair\ split\ ratio\,[\%] = \frac{Number\ of\ hair\ fibers\ with\ closed\ ends}{Total\ number\ of\ hair\ fibers} \cdot 100\%$$

Then the hair was rinsed and shampooed once with Goldwell Dualsenses Scalp Specialist Deep Cleansing Shampoo, analyzed again, and then shampooed for 4 more times. After airdrying the hair, the hair was analyzed by light microscopy and the same calculations from above were performed.

| Split End Recovery Efficacy Test | Inventive | Comparative |
|---|---|---|
| Instant Effect | 82.20% | 20.00% |
| after water rinsing (based on fibers with an instant effect) | 85.00% | 0% |
| after shampoo rinsing (based on fibers with an instant effect) | 80.00% | 0% |

As a result, hair treated with the inventive composition had a high recovery rate from split ends. Moreover, the effect sustained after rinsing with water and shampooing. Consequently the inventive composition had a beneficial effect superior to the comparative example.

Similar results were observed with the following examples. The preparation of the compositions as well as the effect analysis were performed as outlined above.

**Example 2**

| Ingredients | % by weight |
|---|---|
| Cyclopentasiloxane | 20.0 |
| DC FA 4001 CM (INCI: cyclopentasiloxane and acrylates/ polytrimethylsiloxymethacrylate copolymer) | 3.5 |
| Ethylene/Propylene/Styrene Copolymer | 2.0 |
| Butylene/Ethylene/Styrene Copolymer | 2.0 |
| Fragrance | 0.5 |
| Hydrogenated Polyisobutene | Ad 100.00 |

**Example 3**

| Ingredients | % by weight |
|---|---|
| Cyclopentasiloxane | 5.0 |
| Isododecane | 5.0 |
| DC FA 4001 CM | 3.5 |
| Ethylene/Propylene/Styrene Copolymer | 2.0 |
| Butylene/Ethylene/Styrene Copolymer | 2.0 |
| Fragrance | 0.5 |
| Hydrogenated Polyisobutene | Ad 100.00 |

**Example 4**

| Ingredients | % by weight |
|---|---|
| Dimethylsiloxane | 5.0 |
| DC FA 4003 DM (INCI: polydimethylsiloxane and acrylates/ polytrimethylsiloxymethacrylate copolymer) | 3.5 |
| Ethylene/Propylene/Styrene Copolymer | 3.0 |
| Butylene/Ethylene/Styrene Copolymer | 3.0 |
| Fragrance | 0.5 |
| Hydrogenated Polyisobutene | Ad 100.00 |

**Example 5**

| Ingredients | % by weight |
|---|---|
| Isododecane | 35.0 |
| DC FA 4002 ID | 3.5 |
| Ethylene/Propylene/Styrene Copolymer | 3.0 |
| Butylene/Ethylene/Styrene Copolymer | 3.0 |
| Fragrance | 0.5 |
| Hydrogenated Polyisobutene | Ad 100.00 |

**Example 6**

| Ingredients | % by weight |
|---|---|
| Isododecane | 25.0 |
| DC FA 4002 ID | 3.5 |
| Ethylene/Propylene/Styrene Copolymer | 3.0 |
| Butylene/Ethylene/Styrene Copolymer | 3.0 |

(continued)

| Ingredients | % by weight |
|---|---|
| Fragrance | 0.5 |
| Hydrogenated Polyisobutene | Ad 100.00 |

**Examples 7 to 13**

| Ingredients | Example 7 % by weight | Example 8 % by weight | Example 9 % by weight | Example 10 % by weight | Example 11 % by weight | Example 12 % by weight | Example 13 % by weight |
|---|---|---|---|---|---|---|---|
| **Isododecane** | 20.0 | 20.0 | 20.0 | 20.0 | 20.0 | 20.0 | 20.0 |
| **DC FA 4002 ID** | 3.5 | 3.5 | 3.5 | 3.5 | 3.5 | 3.5 | 3.5 |
| **Ethylene/Propylene/ Styrene Copolymer** | 2.0 | 2.0 | 2.0 | 2.0 | 2.0 | 2.0 | 2.0 |
| **Butylene/Ethylene/ Styrene Copolymer** | 2.0 | 2.0 | 2.0 | 2.0 | 2.0 | 2.0 | 2.0 |
| **Fragrance** | 0.5 | 0.5 | 0.5 | 0.5 | 0.5 | 0.5 | 0.5 |
| **Jojoba oil** | 1.0 | 0.2 | - | - | - | - | 1.0 |
| **Olive oil** | 0.2 | 0.6 | - | - | - | - | - |
| **Avocado oil** | 0.2 | 0.6 | - | - | - | - | - |
| **Ubiquinone** | - | - | 2.0 | 2.0 | - | - | - |
| **Cetyl-PG-hydroxyethylpalmitamide** | - | - | - | 0.5 | - | - | 0.5 |
| **2-phenoxyethanol** | - | - | - | - | 2.5 | 2.5 | - |
| **Ethanol** | - | - | - | - | 1.0 | 1.0 | - |
| **4-methocinnamic acid** | - | - | - | - | - | 0.25 | 0.25 |
| **Hydrogenated polyisobutene** | Ad 100.0 | | | | | | |

## Claims

1. An anhydrous cosmetic composition comprising:

   a) one or more silicone dendrimers with at least one group that reacts via free radical polymerization,
   b) One or more monomeric alkanes with a carbon chain length of at least $C_{10}$ or a siloxane, or mixtures of aforementioned monomeric alkanes with siloxans,
   c) One or more homopolymeric olefins, and
   d) One or more copolymers consisting of olefin and aromatic monomers.

2. The composition according to claim 1 **characterized in that** the one group that reacts via free radical polymerization is selected from acryl, methacryl, or vinyl.

3. The composition according to claims 1 and/or 2 **characterized in that** the total concentration by weight of the silicone dendrimer is from 0.01 to 10%, preferably from 0.1% to 5%, more preferably from 0.15% to 2.5%, and most preferably from 0.2% to 2%, calculated to the total of the composition.

4. The composition according to any of the preceding claims **characterized in that** it comprises one or more linear or branched alkanes preferably selected from decane, isodecane, undecane, dodecane, isododecane, tridecane, tetradecane, wherein most preferably it is isododecane.

5. The cosmetic composition according to any of the preceding claims **characterized in that** the siloxane is cyclic or linear or a mixture of linear and/or cyclic siloxanes, wherein the most preferred cyclic siloxan is cyclopentasiloxane and the most preferred linear siloxan is polydimethylsiloxane.

6. The composition according to any of the preceding claims **characterized in that** the component b is comprised at a total concentration in the range of 1% to 40%, preferably from 1% to 20%, by weight calculated to the total of the composition

7. The composition according to any of the preceding claims **characterized in that** the one or more homopolymeric olefin monomer(s) is/ are selected from ethene, propene, isopropene, butene, isobutene, pentene, isopentene, hexane, and isohexene, wherein the most preferred olefin unit is isobutene.

8. The composition according to any of the preceding claims **characterized in that** the total concentration by weight of the homopolymeric olefin is from 50% to 90%, calculated to the total of the composition.

9. The composition according to any of the preceding claims **characterized in that** the olefin copolymer monomers are selected from ethylene, propylene, butylene, pentylene, and hexylene and the aromatic copolymer units are selected from styrene wherein at least one olefinic copolymer unit is polymerized with one or more aromatic unit, selected from styrene.

10. The composition according to any of the preceding claims **characterized in that** the total concentration by weight of the olefinic copolymer is from 0.01% to 7.5%, preferably from 0.1% to 5%, more preferably from 0.25% to 2.5%, calculated to the total of the composition.

11. The composition of any of the preceding claims **characterized in that** it comprises one or more ingredients selected from fragrance, preservatives, antioxidants, essential oils, vegetable oils, organic solvents, ceramides, ubiquinones, UV filtering compounds.

12. The composition according to the any of the preceding claims **characterized in that** it exhibits a viscosity of 1 to 1000 mPas measured with a Brookfield viscosimeter at 25°C.

13. Use of the composition according to any of the preceding claims for reducing hair split ends.

14. Process for treating hair comprising the steps of:

   a) Optionally washing the hair with a cleansing composition,
   b) Applying the composition according to the claims 1 to 12 to hair,

c) Optionally elevating the temperature of the hair from 45°C to 90°C for less than 1 min, preferentially from 10 to 30 s, wherein the temperature is measured at the surface of the hair fibers,
d) Optionally rinsing the hair with water.

**15.** Kit comprising two or more products comprising the composition according to claims 1 to 12.

**Patentansprüche**

**1.** Wasserfreie kosmetische Zusammensetzung, aufweisend:

a) ein oder mehrere Silikon-Dendrimere mit mindestens einer Gruppe, die über freie radikalische Polymerisation reagiert,
b) ein oder mehrere monomere Alkane mit einer Kohlenstoffkettenlänge von mindestens $C_{10}$ oder ein Siloxan oder Mischungen der vorstehend genannten Alkane mit Siloxanen,
c) ein oder mehrere homopolymere Olefine, und
d) ein oder mehrere Copolymere, die aus Olefin und aromatischen Monomeren bestehen.

**2.** Zusammensetzung nach Anspruch 1, **dadurch gekennzeichnet, dass** die eine Gruppe, die über freie radikalische Polymerisation reagiert, aus Acryl, Methacryl oder Vinyl ausgewählt ist.

**3.** Zusammensetzung nach Anspruch 1 und/oder 2, **dadurch gekennzeichnet, dass** die Gesamtkonzentration des Silikon-Dendrimers 0,01 Gewichts-% bis 10 Gewichts-%, vorzugsweise 0,1 Gewichts-% bis 5 Gewichts-%, insbesondere 0,15 Gewichts-% bis 2,5 Gewichts-% und am meisten bevorzugt 0,2 Gewichts-% bis 2 Gewichts-% beträgt, bezogen auf die Gesamtzusammensetzung.

**4.** Zusammensetzung nach einem der vorhergehenden Ansprüche, **dadurch gekennzeichnet, dass** sie ein oder mehrere lineare oder verzweigte Alkane aufweist, vorzugsweise ausgewählt aus Decan, Isodecan, Undecan, Dodecan, Isododecan, Tridecan, Tetradecan, wobei Isododecan am meisten bevorzugt wird.

**5.** Kosmetische Zusammensetzung nach einem der vorhergehenden Ansprüche, **dadurch gekennzeichnet, dass** das Siloxan cyclisch oder linear oder eine Mischung aus linearen und/oder cyclischen Siloxanen ist, wobei das am meisten bevorzugte cyclische Siloxan Cyclopentasiloxan ist und das am meisten bevorzugte lineare Siloxan Polydimethylsiloxan ist.

**6.** Zusammensetzung nach einem der vorhergehenden Ansprüche, **dadurch gekennzeichnet, dass** die Komponente b in einer Gesamtkonzentration im Bereich von 1 Gewichts-% bis 40 Gewichts-%, vorzugsweise von 1 Gewichts-% bis 20 Gewichts-% enthalten ist, bezogen auf die Gesamtzusammensetzung.

**7.** Zusammensetzung nach einem der vorhergehenden Ansprüche, **dadurch gekennzeichnet, dass** das eine oder die mehreren homopolymeren Olefinmonomere aus Ethen, Propen, Isopropen, Buten, Isobuten, Penten, Isopenten, Hexan und Isohexen ausgewählt sind, wobei die am meisten bevorzugte Olefineinheit Isobuten ist.

**8.** Zusammensetzung nach einem der vorhergehenden Ansprüche, **dadurch gekennzeichnet, dass** die Gesamtkonzentration des homopolymeren Olefins 50 Gewichts-% bis 90 Gewichts-% beträgt, bezogen auf die Gesamtzusammensetzung.

**9.** Zusammensetzung nach einem der vorhergehenden Ansprüche, **dadurch gekennzeichnet, dass** die Monomere des Olefin-Copolymers aus Ethylen, Propylen, Butylen, Pentylen und Hexylen ausgewählt sind und die aromatischen Copolymer-Einheiten aus Styrol ausgewählt sind, wobei mindestens eine olefinische Copolymer-Einheit mit einer oder mehreren aromatischen Einheiten polymerisiert wird, ausgewählt aus Styrol.

**10.** Zusammensetzung nach einem der vorhergehenden Ansprüche, **dadurch gekennzeichnet, dass** die Gesamtkonzentration des olefinischen Copolymers 0,01 Gewichts-% bis 7,5 Gewichts-%, vorzugsweise 0,1 Gewichts-% bis 5 Gewichts-%, insbesondere 0,25 Gewichts-% bis 2,5 Gewichts-% beträgt, bezogen auf die Gesamtzusammensetzung.

**11.** Zusammensetzung nach einem der vorhergehenden Ansprüche, **dadurch gekennzeichnet, dass** sie einen oder

mehrere Inhaltsstoffe aufweist, ausgewählt aus einem Duftstoff, Konservierungsmitteln, Antioxidationsmitteln, essentiellen Ölen, pflanzlichen Ölen, organischen Lösungsmitteln, Ceramiden, Ubichinonen, UV-filternden Verbindungen.

**12.** Zusammensetzung nach einem der vorhergehenden Ansprüche, **dadurch gekennzeichnet, dass** sie eine Viskosität von 1 mPas bis 1.000 mPas aufweist, gemessen mit einem Brookfield-Viskosimeter bei 25 °C.

**13.** Verwendung der Zusammensetzung nach einem der vorhergehenden Ansprüche zum Verringern des Spaltens von Haarspitzen.

**14.** Verfahren zum Behandeln von Haaren, aufweisend die Schritte:

a) gegebenenfalls Waschen der Haare mit einer Reinigungszusam m ensetzung,
b) Aufbringen der Zusammensetzung nach Anspruch 1 bis 12 auf die Haare,
c) gegebenenfalls Erhöhen der Temperatur der Haare von 45 °C auf 90 °C für weniger als 1 min, vorzugsweise 10 s bis 30 s, wobei die Temperatur an der Oberfläche der Haarfasern gemessen wird,
d) gegebenenfalls Ausspülen der Haare mit Wasser.

**15.** Kit, welches zwei oder mehr Produkte aufweist, welche die Zusammensetzung nach Anspruch 1 bis 12 aufweisen.

**Revendications**

**1.** Composition cosmétique anhydre comprenant :

a) un ou plusieurs dendrimères de silicone avec au moins un groupe qui réagit via une polymérisation par radical libre,
b) un ou plusieurs alcanes monomériques avec une longueur de chaîne carbonée d'au moins $C_{10}$ ou un siloxane, ou des mélanges des alcanes monomériques mentionnés précédemment avec des siloxanes,
c) une ou plusieurs oléfines homopolymériques, et
d) un ou plusieurs copolymères constitués d'une oléfine et de monomères aromatiques.

**2.** Composition selon la revendication 1, **caractérisée en ce que** le groupe qui réagit via la polymérisation par radical libre est choisi parmi un groupe acrylique, méthacrylique, ou vinylique.

**3.** Composition selon les revendications 1 et/ou 2, **caractérisée en ce que** la concentration totale en poids du dendrimère de silicone est de 0,01 à 10 %, de préférence de 0,1 % à 5 %, plus préférentiellement de 0,15 % à 2,5 %, et idéalement de 0,2 % à 2 %, calculée par rapport au total de la composition.

**4.** Composition selon l'une quelconque des revendications précédentes, **caractérisée en ce qu'**elle comprend un ou plusieurs alcanes linéaires ou ramifiés choisis de préférence parmi le décane, l'isodécane, l'undécane, le dodécane, l'isododécane, le tridécane, le tétradécane, dans laquelle c'est idéalement l'isododécane.

**5.** Composition cosmétique selon l'une quelconque des revendications précédentes, **caractérisée en ce que** le siloxane est cyclique ou linéaire ou un mélange de siloxanes linéaires et/ou cycliques, le siloxane cyclique idéal étant le cyclopenta siloxane et le siloxane linéaire idéal étant le polydiméthyl siloxane.

**6.** Composition selon l'une quelconque des revendications précédentes, **caractérisée en ce que** le composant b est compris dans une concentration totale dans la plage de 1 % à 40 %, de préférence de 1 % à 20 %, en poids calculée par rapport au total de la composition

**7.** Composition selon l'une quelconque des revendications précédentes, **caractérisée en ce que** le ou les monomère(s) d'oléfine homopolymérique(s) est/ sont choisi(s) parmi l'éthène, le propène, l'isopropène, le butène, l'isobutène, le pentène, l'isopentène, l'hexane et l'isohexène, l'unité oléfine idéale étant l'isobutène.

**8.** Composition selon l'une quelconque des revendications précédentes, **caractérisée en ce que** la concentration totale en poids de l'oléfine homopolymérique est de 50 % à 90%, calculée par rapport au total de la composition.

**9.** Composition selon l'une quelconque des revendications précédentes, **caractérisée en ce que** les monomères d'oléfine du copolymère sont choisis parmi l'éthylène, le propylène, le butylène, le pentylène et l'hexylène, et les unités aromatiques du copolymère sont choisies parmi le styrène, où au moins une unité oléfinique du copolymère est polymérisée avec une ou plusieurs unités aromatiques, choisies parmi le styrène.

**10.** Composition selon l'une quelconque des revendications précédentes, **caractérisée en ce que** la concentration totale en poids du copolymère oléfinique est de 0,01 % à 7,5 %, de préférence de 0,1 % à 5 %, plus préférentiellement de 0,25 % à 2,5 %, calculée par rapport au total de la composition.

**11.** Composition selon l'une quelconque des revendications précédentes, **caractérisée en ce qu'**elle comprend un ou plusieurs constituants choisis parmi un parfum, des conservateurs, des antioxydants, des huiles essentielles, des huiles végétales, des solvants organiques, des céramides, des ubiquinones, des composants filtrant les UV.

**12.** Composition selon l'une quelconque des revendications précédentes, **caractérisée en ce qu'**elle présente une viscosité de 1 à 1.000 mPa mesurée avec un viscosimètre de Brookfield à 25 °C.

**13.** Utilisation de la composition selon l'une quelconque des revendications précédentes pour réduire les fourches des cheveux.

**14.** Procédé de traitement des cheveux comprenant les étapes :

a) de lavage optionnel des cheveux avec une composition de nettoyage,
b) d'application de la composition selon les revendications 1 à 12 sur les cheveux,
c) d'élévation optionnelle de la température des cheveux de 45 °C à 90 °C pendant moins de 1 min, de préférence de 10 à 30 s, la température étant mesurée au niveau de la surface des fibres capillaires,
d) de rinçage optionnel des cheveux avec de l'eau.

**15.** Kit comprenant deux ou plusieurs produits comprenant la composition selon les revendications 1 à 12.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0963751 A, Dow Corning Inc.
- EP 1862162 A
- US 20130224130 A
- EP 3009466 A
- WO 201365766 A
- EP 1055674 A


### Non-patent literature cited in the description


- **KIM ; HONG.** *Molecules,* 2009, vol. 14, 3719-3730